# EUROPÄISCHE PATENTSCHRIFT

(11) **EP 1 199 296 B1**
(45) Veröffentlichungstag und Bekanntmachung des Hinweises auf die Patenterteilung: **26.07.2006**
(21) Anmeldenummer: 01122187.6
(22) Anmeldetag: 17.09.2001
(51) Int. Cl.: C07C 41/06, C07C 41/42, C07C 7/148, C07C 43/04, B01D 3/00

(54) **Verfahren zur Herstellung von hochreinem Raffinat II und Methyl-tert.-butylether**
Process for the preparation of highly pure raffinate II and methyl tert-butyl ether
Procédé pour la préparation de raffinat II et de méthyl tert-butyl éther de haute pureté

(30) Priorität: 19.10.2000 DE 10051812; 18.01.2001 DE 10102082
(43) Veröffentlichungstag der Anmeldung: 24.04.2002
(73) Patentinhaber: Oxeno Olefinchemie GmbH, 45772 Marl (DE)
(72) Erfinder: Grund, Gerda. Dr., 48249 Dülmen (DE); Rix, Armin, Dr., 45770 Marl (DE); Büschken, Wilfried, Dr., 45721 Haltern (DE)

(56) Entgegenhaltungen:
- WO-A-94/15894
- US-A- 4 444 648
- US-A- 4 503 265
- US-A- 4 570 026
- US-A- 4 797 133
- US-A- 5 368 691

## Beschreibung

Die Erfindung betrifft ein Verfahren zur Herstellung von hochreinem Raffinat II (einem C₄-Kohlenwasserstoffgemisch) mit einem geringen Isobuten-Gehalt, das sich insbesondere zur Herstellung von reinem 1-Buten und Methyl-tert.-butylether (MTBE) eignet.

Isobuten-freie Butengemische sind geeignet zur Herstellung von hochreinem 1-Buten und/oder zur Herstellung von Butenoligomeren mit geringen Verzweigungsgraden. MTBE ist eine gefragte Vergaserkraftstoffkomponente zur Erhöhung der Octanzahl. Für diesen Zweck ist es unschädlich, dass MTBE auch andere Ether wie Methyl-sec.-butylether oder Oligomere der C₄-Olefine enthält. Hochreines MTBE, das als Lösemittel Verwendung findet, erfordert hinsichtlich der obengenannten Begleitstoffe deutlich engere Grenzen.

MTBE und lineare Butene werden aus C₄-Olefingemischen, beispielsweise dem C₄-Schnitt aus Steamcrackern oder FCC-Einheiten, gewonnen. Diese Gemische bestehen im Wesentlichen aus Butadien, den Monoolefinen, Isobuten, 1-Buten und den beiden 2-Butenen sowie den gesättigten Kohlenwasserstoffen Isobutan und n-Butan. Übliche weltweit ausgeübte Aufarbeitungsverfahren für solche C₄-Schnitte umfassen folgende Schritte: Zuerst wird der größte Teil des Butadiens entfernt. Kann Butadien gut vermarktet werden oder besteht ein Eigenverbrauch, wird es z. B. durch Extraktion oder Extraktionsdestillation abgetrennt. In anderem Fall wird es bis zu Konzentrationen von 1 bis 0,1 % selektiv zu linearen Butenen hydriert. Zurück bleibt in beiden Fällen ein Kohlenwasserstoffgemisch (entsprechend Raffinat I bzw. hydriertes Crack-C₄), das neben den gesättigten Kohlenwasserstoffen (n-Butan und Isobutan) die Olefine (Isobuten, 1-Buten und 2-Butene) enthält. Ein möglicher Weg, das Isobuten aus diesem Gemisch zu entfernen, ist die Reaktion mit Methanol zu MTBE. Zurück bleiben die gesättigten Kohlenwasserstoffe, lineare Butene und gegebenenfalls eine Isobutenrestmenge. Das nach Entfernen des Butadiens und Isobuten erhaltene C₄-Gemisch wird als Raffinat II bezeichnet.

Je nach Weiterverwendung beider Ströme (MTBE und das Olefingemisch, Raffinat II) sind besondere Qualitäten dieser Ströme von entscheidendem Interesse. Wenn das Isobuten aus dem C₄-Schnitt zur Herstellung von Vergaserkraftstoffkomponenten (sei es als MTBE oder Oligomerisat) genutzt wird, bestehen nur geringe Anforderungen hinsichtlich der Reinheit des MTBE. Das MTBE kann andere Ether wie Methyl-sec.-butylether und/oder C₄-Olefinoligomere enthalten.

Raffinat II kann neben den linearen Olefinen größere Anteile an Isobuten enthalten, wenn dieses C₄-Gemisch beispielsweise an sauren Katalysatoren zu möglichst verzweigten C₄-Oligomeren, insbesondere C₈- und C₁₂-Oligomeren umgesetzt wird. Dieses Gemisch ergibt nach Hydrierung eine hochoktanige Vergaserkraftstoffkomponente.

Soll das MTBE, z. B. als reines Lösemittel oder zur Herstellung von hochreinem Isobuten in einer Spaltungsreaktion genutzt werden, darf es nur geringe Mengen an Begleitstoffen enthalten. Die Synthese zum MTBE muss also sehr selektiv durchgeführt werden.

Wenn das Raffinat II zur Herstellung von Oligomeren mit niedrigen Isoindices, d. h. geringem Verzweigungsgrad genutzt werden soll, muss der Gehalt an Isobuten sehr gering sein, vorzugsweise kleiner 1000 wppm. Praktisch Isobuten-freies Raffinat II ist erforderlich, wenn aus diesem Raffinat II reines 1-Buten gewonnen werden soll. Die Konzentration des Isobutens im Raffinat II sollte dann einen Wert von 450 wppm nicht überschreiten. Da die Siedetemperaturdifferenz zwischen Isobuten und 1-Buten lediglich 0,6 °C beträgt, ist eine wirtschaftliche destillative Trennung der beiden Komponenten nicht möglich. In diesem Fall muss Isobuten in der MTBE-Synthese nahezu vollständig umgesetzt werden.

Die höchsten Ansprüche für die MTBE-Synthese liegen vor, wenn MTBE in Lösemittelqualität erzeugt und gleichzeitig das Raffinat II zur 1-Buten-Herstellung verwendet werden soll. Hier ist sowohl ein sehr hoher Isobuten-Umsatz und als auch eine sehr hohe MTBE-Selektivität erforderlich.

Für die Herstellung von MTBE aus Isobuten-haltigen C₄-Kohlenwasserstoffgemischen wie beispielsweise Raffinat I oder hydriertem Crack-C₄ mit Methanol werden in der Technik häufig saure Ionenaustauscherharze (Sulfonsäuregruppen) als heterogene Katalysatoren eingesetzt. Die Umsetzung erfolgt in einem oder mehreren hintereinandergeschalteten Reaktoren, wobei der Katalysator vorzugsweise im Festbett angeordnet ist. Es entsteht ein Produkt, in dem Methanol, Isobuten und MTBE im Gleichgewicht stehen. In jedem Reaktor stellt sich entsprechend den Reaktionsbedingungen (Temperatur, Methanol-Überschuss etc.) der Gleichgewichtsumsatz ein. Dies bedeutet, dass bei dem in technischen Verfahren üblicherweise eingestellten Reaktionsbedingungen ca. 96 % des eingesetzten Isobutens umgesetzt wird. Dieses Gemisch kann anschließend destillativ in eine Sumpffraktion, die MTBE enthält, und eine Kopffraktion, die C₄-Kohlenwasserstoffe und Methanol beinhaltet, getrennt werden. Nach Abtrennung des azeotrop gebundenen Methanols, ist das so hergestellte Raffinat II wegen seines hohen Isobutenrestgehalts für die Herstellung von reinem 1-Buten nicht geeignet.

Um einen praktisch vollständigen Isobuten-Umsatz zu erhalten, werden in der Technik Reaktivdestillationskolonnen eingesetzt. Das sind Kolonnen, die sowohl Trennböden (bzw. Gewebepackungen) und Katalysatoren, auf Trennböden oder in anderen Einbauten oder Gewebepackungen integriert, enthalten. In ihnen erfolgt gleichzeitig die Umsetzung des restlichen Isobutens mit Methanol zu MTBE und die destillative Trennung der Produkte. In eine derartige Kolonne kann auch das Einsatzolefingemisch, beispielsweise Raffinat I oder selektivhydriertes Crack-C₄ eingespeist werden. Besonders geeignet ist der Einsatz dieser Kolonnen für das oben erwähnte Gleichgewichtsgemisch zwecks Erzielung sehr hoher Umsätze. Als Produkte fallen am Kopf ein Azeotrop, bestehend aus Methanol und C₄-Kohlenwasserstoffen, das im Falle der 1-Buten Gewinnung praktisch Isobuten-frei sein muss, und im Sumpf MTBE an.

In US 4 504 687 wird ein Verfahren zur Herstellung von MTBE und einem Isobuten-armen C₄-Strom beschrieben. Dabei erfolgt die Umsetzung eines C₄-Stroms, der sowohl Isobuten als auch lineare Butene enthält, mit Methanol in einer Reaktivdestillationskolonne, in der, durch konstruktive Maßnahmen ermöglicht, Reaktion und Destillation bei verschiedenen Drücken durchgeführt werden. Die Trennung der Kolonne bezüglich des Drucks in einen Destillations- und einen Reaktionsteil ist konstruktiv aufwendig. Über die Reinheit der nach US 4 504 687 hergestellten Produkte werden keine Aussagen gemacht. Für die Reaktivdestillationskolonne wird ein großes Rücklaufverhältnis von 0,5 bis 20 : 1 offenbart.

In US 5 120 403 wird die gleiche Reaktion in einer Reaktivdestillationskolonne, in der der Katalysator geflutet ist, durchgeführt. In einer Flüssigphase kann zwar die Reaktion zu MTBE verbessert ablaufen, jedoch wird so die Destillation erschwert, wodurch die Trennung der Komponenten zur Herstellung von hochreinen Produkten nicht gegeben ist.

In EP 0 885 866 A1 wird ein Verfahren in 6 Ausführungsformen zur Herstellung von MTBE und einem Isobuten-armen C₄-Strom durch Umsetzung eines C₄-Kohlenwasserstoffstroms, der Isobuten und n-Butene enthält, mit Methanol offenbart. Das gemeinsame Merkmal aller Ausführungsformen ist, das zumindest ein Vorreaktor, eine Reaktivdestillationskolonne und ein Nachreaktor in Reihe geschaltet sind.

In allen drei oben genannten Druckschriften werden weder die Qualität des hergestellten MTBE noch der Isobutengehalt des Rest-C₄-Stroms offengelegt.

US 5 368 691 beschreibt die Umsetzung eines C₄-Kohlenwasserstoffgemisches, das Isobuten und lineare Butene enthält, mit Methanol zu MTBE und zu einem C₄-Strom mit den linearen Butenen in einer Reaktivdestillationskolonne. Dabei wird MTBE als Sumpfprodukt in einer Reinheit größer 98 % gewonnen, was den Anforderungen zur Herstellung von MTBE in Lösemittelqualität nicht genügt. Das Beispiel beschreibt ein Kopfprodukt, mit einem Isobuten-Restgehalt von 1,4 %. Zur weiteren Verarbeitung zwecks Herstellung von reinem 1-Buten ist dieser Isobuten-Anteil viel zu hoch. Das Rücklaufverhältnis der Kolonne wird mit 0,5 : 1 bis 5 : 1 angegeben.

Ein weiteres Verfahren zur Herstellung von MTBE und von einem Isobuten-armen C₄-Strom unter Verwendung einer Reaktivdestillationskolonne ist aus US 4 475 005 bekannt. Dabei wird die Kolonne mit einem Rücklaufverhältnis von 1 betrieben. Der Isobutengehalt im Destillat beträgt 4830 wppm und ist damit für eine Weiterverwendung für die Herstellung von reinem 1-Buten deutlich zu hoch.

Da die bekannten Verfahren hinsichtlich des Isobutengehalts im Kopfprodukt bzw. im daraus hergestellten Raffinat II und/oder der Qualität des gewonnenen MTBE und/oder Investment und/oder Energieaufwands nicht überzeugen, bestand die Aufgabe, ein Verfahren zu entwickeln, das ein Raffinat II erzeugt, das für eine kostengünstige Herstellung von 1-Buten geeignet ist und gleichzeitig MTBE in Lösemittelqualität liefert.

Es wurde überraschend gefunden, dass bei der sauer katalysierten Umsetzung von Methanol und einem C₄-Olefingemisch in einer zweistufigen Anlage, Reaktivdestillationskolonne als zweite Stufe, ein Gesamtisobuten-Umsatz von über 99,9 % und ein nahezu Isobuten-freies Raffinat II erhalten werden kann, wobei gleichzeitig ein MTBE anfällt, das praktisch keine Verunreinigungen enthält, wenn in der Reaktivdestillationskolonne spezielle Reaktionsbedingungen eingehalten werden, die durch Rücklaufverhältnis, Temperatur und Druck gekennzeichnet sind.

Gegenstand der vorliegenden Erfindung ist ein Verfahren zur Herstellung von Methyl-tert.-butylether (MTBE) und einem nahezu Isobuten-freien C₄-Kohlenwasserstoffgemisch durch Umsetzung eines Isobuten-haltigen C₄-Kohlenwasserstoffstroms mit Methanol an einem sauren Katalysator, wobei in einer ersten Stufe in einem oder mehreren Reaktor(en) Isobuten mit Methanol bis zur Gleichgewichtseinstellung zu MTBE umgesetzt wird und das restliche im Gemisch vorliegende Isobuten in einer zweiten Stufe in einer Reaktivdestillationskolonne, an einem sauren Ionenaustauscherharz umgesetzt wird, wobei die Reaktivdestillationskolonne im Druckbereich von 3 bis 15 bara und im Temperaturbereich der Reaktionszone von 55 °C bis 75 °C bei einem Rücklaufverhältnis kleiner 1 betrieben wird.

Das Kopfprodukt enthält wegen des hohen Umsatzes weniger als 450 wppm Isobuten und ist daher für die Herstellung von reinem 1-Buten hervorragend geeignet. Das hergestellte MTBE besitzt eine Reinheit, die es als Lösemittel verwendbar macht.

Als Rücklaufverhältnis bezeichnet man definitionsgemäß das Verhältnis des Rücklaufstromes in die Kolonne zum abgeführten Destillatstrom.

Dieser Befund, dass bei geringen Rückflussverhältnissen und Temperaturen in den Katalysator-Packungen ein besserer Isobuten-Umsatz erhalten wird als bei höheren Rückflussverhältnissen, ist überraschend, da in der Literatur das Gegenteil beschrieben wird.

Beispielsweise wird in folgenden Druckschriften gezeigt, dass ähnlich wie bei einer Destillation, die Reaktionsrate der Umsetzung der Isobuten-haltigen C₄-Strömen mit Methanol zu MTBE in einer Reaktivdestillationskolonne mit zunehmendem Rücklaufverhältnis ansteigt(Lawrence A. Smith, D.Hearn, Catalytic Destillation, Proc. Intersoc. Energy Convers. Conf. (1984) 19^{th}, (Vol 2), p 998-1002; Miguel A. Isla, Horazio A. Irazoqui, Modeling, Analysis, and Simulation of a Methyl tert-Butyl Ether Reactive Destillation Column, Ind. Eng. Chem. Res. 1966, 35, 2696-2708; Hoshang Subawalla, James R. Fair, Design Guideline for Solid-Catalyzed Distillation Systems, Ind. Eng. Chem. Res. 1999,38, 3696 - 3709) oder in "Rate-Based Modeling of Reactive Destillation Systems", V. Pinjala and T. L. Marker et al., Topical Conference on Separations Technologies ATChE, 1-6, 11. 1992. Es lag daher gegen den Trend der Fachwelt, ein kleines Rücklaufverhältnis einzustellen.

Das erfindungsgemäße Verfahren weist eine Reihe von Vorteilen auf. Mit Hilfe des Verfahrens kann ein Destillat erhalten werden, das mit weniger als 450 wppm Isobuten bezogen auf die C₄-Kohlenwasserstoffe und/oder mit weniger als 0,5 wppm MTBE (ausgenommen das Methanol), sich für die Herstellung von reinem 1-Buten mit weniger als 1000 wppm Isobuten eignet. Das MTBE ist von solch hoher Qualität, das es sowohl als Vorstufe für die Herstellung von hochreinem Isobuten als auch als Lösemittel genutzt werden kann.

Die Absenkung des Rücklaufverhältnisses führt darüber hinaus in signifikante Dampfeinsparung, wodurch das erfindungsgemäße Verfahren einen geringen Energiebedarf besitzt.

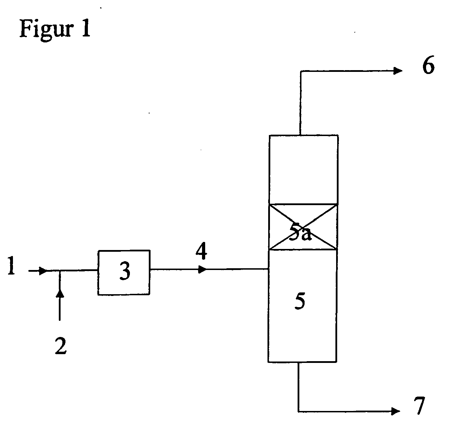

Nach dem erfindungsgemäßen Verfahren wird die Umsetzung von Isobuten mit Methanol zu MTBE in zwei Stufen durchgeführt (siehe Fig. 1). Die erste Stufe beinhaltet die Reaktion von Isobuten im C₄-Gemisch mit Methanol in einem oder mehreren Reaktoren, bis zur Einstellung des thermodynamischen Gleichgewichts aus MTBE, Methanol und Isobuten. Dies liegt in der Regel zwischen 94 und 96 % Isobuten-Umsatz. Die Reaktoren der ersten Stufe können herkömmliche Festbettreaktoren mit den gleichen Katalysatoren sein, die im Folgenden für die zweite Stufe beschrieben werden. Die Reaktoren werden in üblicher Weise bei 30 - 110 °C und 5 - 50 bara betrieben.

Zusammensetzungen der so erhaltenen Reaktionsmischungen sind in den Beispielen beschrieben. In der Regel enthalten diese Gemische unter 1 Gew.-% Isobuten, das in der folgenden zweiten Stufe, der Reaktivdestillationskolonne sehr selektiv zu MTBE umgesetzt wird.

Diese Reaktivdestillationskolonne enthält in der Verstärkersäule den Katalysator, unter- und oberhalb der Katalysatorpackung befinden sich Trennböden oder Destillationspackungen. Der Katalysator ist entweder in einer Packung integriert, beispielsweise KataMax® (EP 0 428 265), KataPak® (EP 0 396 650) oder MultiPak® (Gebrauchsmuster Nr. 298 7 007.3), oder auf Formkörper aufpolymerisiert (US 5 244 929).

Die Zone oberhalb der Katalysatorpackung besteht aus 5 bis 20, insbesondere 10 bis 15 Trennstufen. Die Katalysatorzone lässt sich mit einer destillativen Wirkung von 1 bis 5 theoretischer Trennstufen pro Meter Packungshöhe abschätzen. Die Trennzone unterhalb des Katalysators umfasst 12 bis 36, insbesondere 20 bis 30 Trennstufen.

Als eigentlicher Katalysator wird in beiden Stufen des Verfahrens ein fester Stoff, der weder im Einsatzstoffgemisch noch im Produktgemisch löslich ist, mit sauren Zentren an seiner Oberfläche eingesetzt. Der Katalysator darf unter Reaktionsbedingungen keine sauren Stoffe an das Produktgemisch abgeben, weil dies zu Ausbeuteverlusten führen würde.

Für die Aktivität der Katalysatoren gilt, dass sie unter Reaktionsbedindungen die Addition von Methanol an Isobuten bewirken, jedoch kaum die Addition an lineare Butene. Weiterhin dürfen sie die Oligomerisierung von Olefinen und die Dimethyletherbildung kaum katalysieren.

Eine im Verfahren der Erfindung einsetzbare Gruppe von sauren Katalysatoren sind feste Ionenaustauscherharze mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden. Stark saure Harze des Styrol-Divinyl-Typs werden u. a. unter folgenden Handelsnamen verkauft: Duolite C20, Duolite C26, Amberlyst A15, Amberlyst A35, Amberlite IR-120, Amberlite 200, Dowex 50, Lewatit SPC 118, Lewatit SPC 108, K2611, K2621, OC 1501.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Es werden vorzugsweise makroporöse Harze eingesetzt, wie beispielsweise Lewatit SCP 118, Lewatit SCP 108, Amberlyst A15 oder Amberlyst A35, K2621. Das Porenvolumen beträgt 0,3 bis 0,9 ml/g , insbesondere 0,5 bis 0,9 ml/g. Die Korngröße des Harzes liegt zwischen 0,3 mm und 1,5 mm, insbesondere zwischen 0,5 mm und 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf Lieferform, 0,7 - 2,0 mol/l, insbesondere 1,1-2,0 mol/l.

Als Einsatzstoffe für das erfindungsgemäße Verfahren können C₄-Kohlenwasserstoffgemische, die sowohl Isobuten als auch lineare Butene, jedoch keine Acetylenderivate und weniger als 8000 wppm Butadien enthalten, eingesetzt werden. Technische Gemische, die sowohl Isobuten als auch lineare Butene enthalten können, sind beispielsweise Leichtbenzinfraktionen aus Raffinerien, C₄-Fraktionen aus FCC-Einheiten oder Steamcracker, Gemische aus Fischer-Tropsch-Synthesen, Gemische aus Dehydrierung von Butanen, Gemische aus Skelettisomerisierung linearer Butene, Gemische, entstanden durch Metathese von Olefinen oder anderen technischen Prozessen.

Diese Gemische können nach Entfernung der mehrfach ungesättigten Verbindungen in das erfindungsgemäße Verfahren eingesetzt werden. Beispielsweise kann die Gewinnung eines geeigneten Einsatzstoffsgemisch aus der C₄-Fraktion eines Steamcrackers durch Extraktion des Butadiens oder dessen Selektivhydrierung zu linearen Butenen erfolgen. Dieses Gemisch (Raffinat I bzw. selektivhydriertes Crack-C₄) besteht aus n-Butan, Isobutan, den drei linearen Butenen und Isobuten und ist ein bevorzugtes Edukt für das erfindungsgemäße Verfahren.

Das Einsatzkohlenwasserstoffgemisch kann zusammen mit Methanol in die erste Verfahrensstufe eingespeist werden. Als Katalysatoren werden die gleichen oder ähnliche wie in der Reaktivdestillationskolonne verwendet. Dabei entsteht ein Gemisch, in dem Isobuten, Methanol und MTBE im Gleichgewicht stehen. Eine bevorzugte Ausführung des erfindungsgemäßen Verfahrens ist es, ein Gleichgewichtsgemisch oder ein Gemisch, das in der Nähe des Gleichgewichts liegt, in der ersten Stufe herzustellen und der Reaktivdestillationskolonne (zweite Stufe) zuzuführen.

Im Kolonnenzulauf der zweiten Stufe kann mehr Methanol enthalten sein, als für die vollständige Umsetzung des noch vorhandenen Isobutens gebraucht wird. Der Methanolüberschuss sollte jedoch derart begrenzt werden, dass einerseits eine ausreichende Methanolmenge für das sich bildende Azeotrop aus Methanol und C₄-Kohlenwasserstoffen vorhanden ist, andererseits nicht so viel, dass Methanol ins Sumpfprodukt geriete, so dass ein spezifikationsgerechtes MTBE (Methanolgehalt unter 5000 wppm) erhalten wird.

Optional kann, wenn der Methanolgehalt im Kolonnenzulauf unter dem maximal zulässigen Wert liegt, zusätzliches Methanol vor dem Kolonnenzulauf des Eduktgemisches eingespeist werden. Darüber hinaus kann am Kopf der Reaktivdestillationskolonne über eine gesonderte Einrichtung eine Methanol-Einspeisung erfolgen.

Die Temperatur des Kolonnenzulaufs liegt unabhängig von dessen Zusammensetzung, Reaktionsdruck in der Kolonne und Durchsatz zwischen 50 °C und 80 °C, vorzugsweise zwischen 60 °C und 75 °C.

Die mittlere Temperatur in der Katalysatorzone beträgt abhängig vom Druck in der Kolonne vorzugsweise 55 °C bis 70 °C, besonders bevorzugt 58 °C bis 67 °C.

Die Reaktivdestillationskolonne wird bei Drücken, gemessen am Kolonnenkopf, von 3 - 15, bevorzugt 5 bara bis 9 bara betrieben, insbesondere von 7 bara bis 8,5 bara.

Die hydraulische Belastung in der katalytischen Packung der Kolonne beträgt bevorzugt 10 % bis 110 %, vorzugsweise 20 % bis 70 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastung kennzeichnet die minimale Belastung, unterhalb derer die Trennwirkung absinkt oder zusammenbricht infolge unregelmäßiger Strömung oder Leerlaufen der Kolonne - z. B. der Böden. (Vauck/Müller, "Grundoperationen chemischer Verfahrenstechnik", S. 626, VEB Deutscher Verlag für Grundstoffindustrie.)

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag1991).

Im erfindungsgemäßen Verfahren wird die Kolonne mit Rücklaufverhältnissen kleiner als 1 betrieben, insbesondere mit solchen, die größer 0,6 und kleiner 1, bevorzugt zwischen 0,7 und 0,9 liegen.

Mit diesen Rücklaufsverhältnissen werden erfindungsgemäß Isobuten-Restkonzentrationen im Raffinat II von kleiner 450, bevorzugt 400, ganz besonders kleiner 300 wppm (bezogen auf das C₄-Gemisch im Destillat) erhalten. Das optimale Rücklaufverhältnis hängt vom Durchsatz, Zusammensetzung des Kolonnenzulaufs und dem Kolonnendruck ab. Es liegt jedoch immer in den oben genannten Bereichen.

Optional kann in der zweiten Stufe des Verfahrens ein Kopfprodukt, enthaltend ein C₄-Kohlenwasserstoffgemisch und Methanol, mit einem Isobuten-Gehalt von weniger als 450, bevorzugt weniger als 400, ganz besonders bevorzugt weniger als 300 wppm sowie ein Sumpfprodukt, enthaltend MTBE mit einem Gehalt von Methyl-sec.-butylether (MSBE) von weniger als 2500 wppm erhalten werden.

Dieses Kopfprodukt kann wiederum in ein C₄-Kohlenwasserstoffgemisch und Methanol getrennt werden, wobei das C₄-Kohlenwasserstoffgemisch weniger als 0,5 wppm MTBE und/oder TBA enthält.

Das Sumpfprodukt der Reaktivdestillationskolonne besteht bevorzugt aus MTBE mit weniger als 2500 wppm Methyl-sec.-butylether und weniger als 2500 wppm C₈-Kohlenwasserstoffen. Eine weitere Reinigung des MTBE ist nicht mehr erforderlich, wenn es als Komponente für Ottokraftstoffe eingesetzt wird.

Aus dem Kopfprodukt kann z. B. durch Extraktion mit Wasser das Methanol abgetrennt werden. Aus dem so erhaltenen Raffinat II können Spuren von Butadien durch Selektivhydrierung (SHP) entfernt werden. Dieses Gemisch kann destillativ in 1-Buten, Isobutan und ein Gemisch aus 2-Butenen, n-Butan oder in ein 1-Buten, 2-Buten und n-Butan getrennt werden.

Das derart hergestellte, reine 1-Buten enthält weniger als 1000 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd.

Eine weitere Verwendung des erfindungsgemäß hergestellten nahezu Isobuten-freien, Raffinats II ist die Herstellung von n-Buten-Oligomeren, insbesondere nach dem Octol-Prozess.

Die nach Abtrennung bzw. Umsetzung der linearen Butene aus dem Raffinat II zurückbleibenden Kohlenwasserstoffe können gegebenenfalls nach Hydrierung (CSP) zu Isobutan und n-Butan aufgearbeitet werden.

Der in der Reaktivdestillationskolonne als Sumpfprodukt anfallende MTBE kann für verschiedene Zwecke genutzt werden. Da er nur äußerst geringe Mengen an Methyl-sec.-butylether (MSBE) enthält, ist er für die Herstellung von hochreinem Isobuten durch seine Rückspaltung geeignet, da praktisch keine linearen Butene (durch Rückspaltung des Methyl-sec.-butylethers) entstehen können. Die MTBE-Spaltung kann z. B. gemäß DE 100 20 943.2 durchgeführt werden.

Aufgrund des geringen Gehalts an Nebenprodukten (MSBE und C₈-Olefinen) kann der derart gewonnene MTBE nach Abtrennung von den restlichen Alkoholen als Lösemittel in der Analytik oder bei organischen Synthesen verwendet werden.

Weiterhin ist seine Nutzung als Komponente für Ottokraftstoffe möglich.

Ein Blockschema einer Anlage, in der das erfindungsgemäße Verfahren durchgeführt werden kann, ist in Figur 1 dargestellt.

Ein C₄-Kohlenwasserstoffgemisch (Raffinat I oder selektiv hydriertes Crack-C₄) (1) wird mit Methanol (2) im Reaktor (3), der einen saures Ionenaustauscherharz enthält, zu einem MTBE-haltigen Reaktionsgemisch (4) umgesetzt, das in eine Reaktivdestillationskolonne (5) unterhalb der Katalysatorpackung (5a) eingespeist wird. Als Kopfprodukt (6) fällt Methanol und ein C₄-Strom mit weniger als 300 wppm Isobuten an. Als Sumpfprodukt (7) wird MTBE abgezogen.

Die folgenden Beispiele sollen die Erfindung erläutern, ohne die Anwendungsbreite einzuengen, die sich aus den Patentansprüchen ergibt.

### Beispiel 1: (Vergleichsbeispiel)

Die Umsetzung des C₄-Gemisches mit der in Tab.1A angegebenen Zusammensetzung wurde in einer Reaktivdestillationskolonne (s. Fig. 1), die mit Amberlyst A15 in KATAMAX Packung ausgestattet war, durchgeführt. Die Packung wurde im oberen Teil der Kolonne angeordnet. Oberhalb des Zulaufes befinden sich Trennungsböden, folglich drei KATAMAX Packungen mit jeweils einem Flüssigkeitsverteiler, schließlich erneut Destillationsböden. Unterhalb des Zulaufes befinden sich ausreichend dimensionierte Abtriebsteile, um die Trennung des MTBE von C₄-Kohlenwasserstoffen zu bewerkstelligen.

Die Kolonne wurde bei einem Kopfdruck von 8,2 bara, Temperaturen in den Packungen vom Zulaufboden aufwärts gesehen von 65,8 °C, 65,4 °C, 65,1 °C, bei einer hydraulischen Belastung der katalytischen Packung von 36 % und einem Rücklaufverhältnis von 1,02 betrieben. Bei dieser Fahrweise wurde ein Isobuten -Umsatz in der Reaktivdestillationskolonne von 93,4 % erreicht, nicht genug um die erforderliche Isobutenrestkonzentration im Raffinat zu erreichen (siehe Analysen in Tab. 1B)

**Tabelle 1A: Zusammensetzung der Ströme der Reaktivdestillationskolonne**

| | Zulauf (Gew.- %) | Destillat (Gew.-%) | Sumpf (Gew.-%) |
|---|---|---|---|
| C₄-KW Gemisch * | 58,93 | 94,14 | 0,13 |
| MTBE | 37,02 | 0 | 98,00 |
| MSBE | 0,04 | 0 | 0,32 |
| Methanol | 3,68 | 5,86 | 0,57 |
| TBA | 0,29 | 0 | 0,60 |
| C₈ | 0,04 | 0 | 0,27 |
| Rest | 0 | 0 | 0,11 |

**Tabelle 1B: Verteilung der C₄-KW im Gemisch * im Zulauf und Destillat der Kolonne (jeweils normiert auf 100 %)**

| | Zulauf (Gew.- %) | Destillat (Gew.- %) |
|---|---|---|
| iso-Butan | 4,79 | 6,00 |
| n-Butan | 14,46 | 14,32 |
| trans-Buten-2 | 23,66 | 24,56 |
| 1-Buten | 44,92 | 43,335 |
| Isobuten | 0,68 | 0,048 |
| cis-Buten-2 | 10,99 | 11,40 |
| 1,3-Butadien | 0,50 | 0,34 |

### Beispiel 2 gemäß der Erfindung

Die Reaktivdestillationskolonne wurde unter gleichem Druck (8,2 bara), Temperaturen in der Packung von 66,5 °C, 66,2 °C, 65,8 °C, gleicher hydraulischen Belastung in der katalytischen Packung von 36 % und vergleichbaren Zusammensetzung des Zustromes wie im Beispiel 1 betrieben. Geändert wurde das Rücklaufverhältnis in der Kolonnen, welches auf 0,89 abgesenkt wurde. Analog zum Beispiel 1 zeigen Tabellen 2A und 2B die Zusammensetzung der Ströme und lassen den Umsatz und Selektivität ableiten.

**Tabelle 2A: Zusammensetzung der Ströme der Reaktivdestillationskolonne**

| | Zulauf (Gew.- %) | Destillat (Gew.- %) | Sumpf (Gew.- %) |
|---|---|---|---|
| C₄-KW Gemisch * | 61,471 | 93,88 | 0,17 |
| MTBE | 34,82 | 0 | 98,04 |
| MSBE | 0,04 | 0 | 0,31 |
| Methanol | 3,41 | 6,12 | 0,48 |
| TBA | 0,22 | 0 | 0,62 |
| C₈ | 0,039 | 0 | 0,23 |
| Rest | 0 | 0 | 0,15 |

**Tabelle 2B: Verteilung der C₄-KW im Gemisch * im Zulauf und Destillat der Kolonne**

| | Zulauf (Gew.- %) | Destillat (Gew.- %) |
|---|---|---|
| iso-Butan | 5,426 | 5,798 |
| n-Butan | 13,549 | 13,861 |
| trans-Buten-2 | 25,733 | 26,138 |
| 1-Buten | 42,383 | 42,004 |
| Isobuten | 0,720 | 0,018 |
| cis-Buten-2 | 11,880 | 11,906 |
| 1,3-Butadien | 0,309 | 0,275 |

Beispiel 2 zeigt im Vergleich zu Beispiel 1, wie vorteilhaft die Verringerung des Rücklaufverhältnis von 1,02 auf 0,89 ist. Die Isobutenkonzentration im Destillat liegt, bezogen auf die C4-Kohlenwasserstoffe, im Beispiel 2 bei 0,018 %. Dieses Destillat ist im Gegensatz zum Destillat des Beispiels 1 zur Herstellung von 1-Buten mit einem Isobutengehalt von weniger als 1000 wppm geeignet.

### Beispiel 3 (gemäß der Erfindung)

Die Reaktivdestillationskolonne wurde bei einem Druck von 7,4 bara, Temperaturen in den Packungen von 62,2°C, 62,0°C, 61,6°C, bei einer hydraulischen Belastung in der katalytischen Packung von 37 % und einem Rücklaufverhältnis von 0,89 betrieben. Analog zum Beispiel 1 zeigen Tabellen 3A und 3B die Zusammensetzung der Ströme in der Kolonne

**Tabelle 3A: Zusammensetzung der Ströme der Reaktivdestillationskolonne**

| | Zulauf (Gew.- %) | Destillat (Gew.- %) | Sumpf (Gew.- %) |
|---|---|---|---|
| C₄-KW Gemisch* | 61.77 | 95,4 | 0,11 |
| MTBE | 34,84 | 0 | 98,49 |
| MSBE | 0,04 | 0 | 0,21 |
| Methanol | 3,06 | 4,6 | 0,32 |
| TBA | 0,19 | 0 | 0,51 |
| C₈ | 0,04 | 0 | 0,19 |
| Rest | 0,06 | 0 | 0,17 |

**Tabelle 3B: Verteilung der C₄-KW im Gemisch * im Zulauf und Destillat der Kolonne**

| | Zulauf (Gew.- %) | Destillat (Gew.- %) |
|---|---|---|
| iso-Butan | 5,271 | 5,576 |
| n-Butan | 15,088 | 15,350 |
| trans-Buten-2 | 24,589 | 24,335 |
| 1-Buten | 42,185 | 43,107 |
| Isobuten | 0,709 | 0,018 |
| cis-Buten-2 | 11,925 | 11,393 |
| 1,3-Butadien | 0,233 | 0,221 |

Beispiel 3 zeigt, dass bei gleichem Rücklaufverhältnis wie im Beispiel 2 durch Reduzierung der Drücke in den Katalysator-Packungen, die Konzentration von Methyl-sec.-butylether (MSBE) im Sumpfprodukt gesenkt wurde, ohne dass der Isobutengehalt im Destillat ansteigt. Ein Sumpfprodukt dieser Qualität ist für die Herstellung von MTBE in Lösemittelqualität geeignet.

## Patentansprüche

1. Verfahren zur Herstellung von Methyl-tert.-butylether (MTBE) und einem nahezu Isobuten-freien C₄-Kohlenwasserstoffgemisch durch Umsetzung eines Isobutenhaltigen C₄-Kohlenwasserstoffstroms mit Methanol an einem sauren Katalysator,
**dadurch gekennzeichnet,**
**dass** in einer ersten Stufe in einem oder mehreren Reaktoren Isobuten mit Methanol bis zur Gleichgewichtseinstellung zu MTBE umgesetzt wird und das restliche im Gemisch vorliegende Isobuten in einer zweiten Stufe in einer Reaktivdestillationskolonne, an einem sauren Ionenaustauscherharz umgesetzt wird, wobei die Reaktivdestillationskolonne im Druckbereich von 3 bis 15 bara und im Temperaturbereich der Reaktionszone von 55 °C bis 75 °C bei einem Rücklaufverhältnis kleiner 1 betrieben wird.

2. Verfahren nach Anspruch 1,
**dadurch gekennzeichnet,**
**dass** die Reaktivdestillationskolonne mit einem Rücklaufverhältnis von größer 0,6 und kleiner 1 betrieben wird.

3. Verfahren nach einem der Ansprüche 1 oder 2,
**dadurch gekennzeichnet,**
**dass** in einer zweiten Stufe ein Kopfprodukt, enthaltend Methanol und ein C₄₋Kohlenwasserstoffgemisch, mit einem Isobuten-Gehalt von weniger als 450 wppm, bezogen auf das C₄-Kohlenwasserstoffgemisch, erhalten wird.

4. Verfahren nach einem der Ansprüche 1 oder 2,
**dadurch gekennzeichnet,**
**dass** die Temperatur in der Reaktionszone zwischen 55 °C und 70 °C liegt.

5. Verfahren nach einem der Ansprüche 1 bis 3,
**dadurch gekennzeichnet,**
**dass** der Druck 7 bara bis 8,5 bara beträgt.

6. Verfahren nach einem der Ansprüche 1 bis 5,
**dadurch gekennzeichnet,**
**dass** die Reaktivdestillationskolonne mit einer hydraulischen Belastung der katalytischen Packung von 10 % bis 110 % betrieben wird

7. Verfahren nach einem der Ansprüche 1 bis 6,
**dadurch gekennzeichnet,**
**dass** in der zweiten Stufe ein Kopfprodukt, enthaltend ein C₄-Kohlenwasserstoffgemisch und Methanol, mit einem Isobuten-Gehalt von weniger als 450 wppm, bezogen auf das C₄₋Kohlenwasserstoffgemisch, sowie ein Sumpfprodukt, enthaltend MTBE mit einem Gehalt von Methyl-sec.-butylether (MSBE) von weniger als 2500 wppm erhalten wird.

8. Verfahren nach Anspruch 7,
**dadurch gekennzeichnet,**
**dass** das Kopfprodukt in ein C₄-Kohlenwasserstoffgemisch und Methanol getrennt wird.

9. Verfahren nach Anspruch 7 oder 8,
**dadurch gekennzeichnet,**
**dass** das C₄-Kohlenwasserstoffgemisch weniger als 0,5 wppm MBTE enthält.

10. Verfahren nach Anspruch 7 oder 8,
**dadurch gekennzeichnet,**
**dass** das Sumpfprodukt weniger als 2500 wppm MSBE und weniger als 2500 wppm C₈₋Kohlenwasserstoffe enthält.

## Claims

1. A process for preparing methyl tert-butyl ether (MTBE) and a virtually isobutene-free C₄₋hydrocarbon mixture by reaction of an isobutene-containing C₄-hydrocarbon stream with methanol over an acid catalyst, **characterized in that**, in a first stage in one or more reactor(s), isobutene is reacted with methanol to equilibrium formation of MTBE and, in a second stage in a reactive distillation column, the remaining isobutene present in the mixture is reacted over an acid ion exchange resin, the reactive distillation column being operated in a pressure range from 3 to 15 bar abs. and in a temperature range in the reaction zone from 55°C to 75°C at a reflux ratio of less than 1.

2. A process according to claim 1, **characterized in that** the reactive distillation column is operated at a reflux ratio which is greater than 0.6 and less than 1.

3. A process according to either one of claims 1 and 2, **characterized in that**, in a second stage, a top product containing methanol and a C₄-hydrocarbon mixture having an isobutene content of less than 450 ppm by weight, based on the C₄-hydrocarbon mixture, is obtained.

4. A process according to either one of claims 1 and 2, **characterized in that** the temperature in the reaction zone is from 55°C to 70°C.

5. A process according to any one of claims 1 to 3, **characterized in that** the pressure is from 7 bar abs, to 8.5 bar abs.

6. A process according to any one of claims 1 to 5, **characterized in that** the reactive distillation column is operated at a hydraulic loading of the catalytic packing of from 10% to 110%.

7. A process according to any one of claims 1 to 6, **characterized in that**, in the second stage, a top product containing a C₄-hydrocarbon mixture and methanol and having an isobutene content of less than
450 ppm by weight, based on the C₄-hydrocarbon mixture, and a bottom product containing MTBE and having a methyl sec-butyl ether (MSBE) content of less than 2500 ppm by weight are obtained.

8. A process according to claim 7, **characterized in that** the top product is separated into a C₄₋hydrocarbon mixture and methanol.

9. A process according to either of claims 7 and 8, **characterized in that** the C₄-hydrocarbon mixture contains less than 0.5 ppm by weight of MTBE.

10. A process according to either of claims 7 and 8, **characterized in that** the bottom product contains less than 2500 ppm by weight of MSBE and less than 2500 ppm by weight of C₈-hydrocarbon.

## Revendications

1. Procédé de préparation de méthyl tert-butyléther (MTBE) et d'un mélange d'hydrocarbures en C₄ pratiquement exempt d'isobutène par conversion d'un courant d'hydrocarbures en C₄ contenant de l'isobutène avec du méthanol sur un catalyseur acide,
**caractérisé en ce que**
dans une première étape, on convertit de l'isobutène avec du méthanol, dans un ou plusieurs réacteurs, jusqu'à l'obtention d'un équilibre pondéral avec le MTBE et dans une deuxième étape, on convertit l'isobutène restant présent dans le mélange dans une colonne de distillation réactive, sur une résine acide échangeuse d'ions, la colonne de distillation réactive fonctionnant dans un domaine de pression de 3 à 15 bars et dans un domaine de température de la zone de réaction de 55 à 75 °C avec un taux de reflux inférieur à 1.

2. Procédé selon la revendication 1,
**caractérisé en ce que**
la colonne de distillation réactive fonctionne avec un taux de reflux supérieur à 0,6 et inférieur à 1.

3. Procédé selon l'une quelconque des revendications 1 ou 2,
**caractérisé en ce que**
dans une deuxième étape, on obtient un produit de tête, contenant du méthanol et un mélange d'hydrocarbures en C₄, ayant une teneur en isobutène inférieure à 450 ppm en poids par rapport au mélange d'hydrocarbures en C₄.

4. Procédé selon l'une quelconque des revendications 1 ou 2,
**caractérisé en ce que**
la température, dans la zone de réaction, est comprise entre 55 et 70 °C.

5. Procédé selon l'une quelconque des revendications 1 à 3,
**caractérisé en ce que**
la pression est de 7 à 8,5 bars.

6. Procédé selon l'une quelconque des revendications 1 à 5,
**caractérisé en ce que**
la colonne de distillation réactive fonctionne avec une charge hydraulique du garnissage catalytique de 10 à 110 %.

7. Procédé selon l'une quelconque des revendications 1 à 6,
**caractérisé en ce que**
dans la deuxième étape, on obtient un produit de tête contenant un mélange d'hydrocarbures en C₄ et du méthanol, avec une teneur en isobutène inférieure à 450 ppm en poids, par rapport au mélange d'hydrocarbures en C₄, ainsi qu'un produit indistillable, contenant du MTBE avec une teneur en méthyl sec-butyl éther (MSBE) inférieure à 2 500 ppm en poids.

8. Procédé selon la revendication 7,
**caractérisé ce que**
le produit de tête est séparé en un mélange d'hydrocarbures en C₄ et de méthanol.

9. Procédé selon la revendication 7 ou 8
**caractérisé en ce que**
le mélange d'hydrocarbures en C₄ contient moins de 0,5 ppm en poids de MBTE.

10. Procédé selon la revendication 7 ou 8,
**caractérisé en ce que**
le produit indistillable contient moins de 2 500 ppm en poids de MSBE et moins de 2 500 ppm en poids d'hydrocarbures en C₈.
